# EUROPÄISCHE PATENTSCHRIFT

(11) **EP 3 104 917 B1**
(45) Veröffentlichungstag und Bekanntmachung des Hinweises auf die Patenterteilung: **20.04.2022**
(21) Anmeldenummer: 15714163.1
(22) Anmeldetag: 12.02.2015
(51) Int. Cl.: A61M 5/44

(54) **VORRICHTUNG ZUM ERWÄRMEN VON STRÖMENDEN FLUIDEN**
DEVICE FOR HEATING FLOWING FLUIDS
DISPOSITIF DE CHAUFFAGE DE FLUIDES EN ÉCOULEMENT

(30) Priorität: 12.02.2014 DE 102014101749; 11.03.2014 DE 102014103233
(43) Veröffentlichungstag der Anmeldung: 21.12.2016
(73) Patentinhaber: Barkey GmbH & Co. KG, 33818 Leopoldshöhe (DE)
(72) Erfinder: NOWACK, Armin, 33803 Steinhagen (DE); BARKEY, Christian, 33619 Bielefeld (DE); BARKEY, Thomas, 33813 Oerlinghausen (DE); TESCHNER, Holger, 33617 Bielefeld (DE)
(74) Vertreter: Ostermann, Thomas
(86) Internationale Anmeldenummer: PCT/DE2015/100060
(87) Internationale Veröffentlichungsnummer: WO 2015/120843

(56) Entgegenhaltungen:
- US-A1- 2002 081 109
- US-A1- 2002 181 948
- US-A1- 2004 026 068
- US-A1- 2004 190 885
- US-B1- 7 316 666
- None

## Beschreibung

Die Erfindung betrifft eine Vorrichtung zum Erwärmen von strömenden Fluiden nach dem Oberbegriff des Patentanspruchs 1.

Aus der DE 198 28 923 B4 ist eine Vorrichtung zum Erwärmen von strömenden Fluiden bekannt, die ein flächiges Fluidgehäuse mit einem mäanderförmigen Fluidkanal aufweist. Der Fluidkanal wird durch ein rohrförmiges Bauteil gebildet, das von einer Abdeckfolie umgeben ist. An einem umlaufenden Rand ist die Folie verschweißt, so dass sich insgesamt ein flächiges Fluidgehäuse ergibt. Dieser Rand weist Bohrungen auf, mittels derer zu beiden Seiten des flächigen Fluidgehäuses elektrisch betriebene Heizplatten ansetzbar sind zum Erwärmen des durch den Fluidkanal strömenden Fluids. Nachteilig an der bekannten Vorrichtung ist, dass der Bauraumaufwand relativ groß ist, da der Fluidkanal in einer geschlossenen Tasche der Abdeckfolie angeordnet ist, wobei ein Zwischenraum zwischen dem Fluidkanal und dem umgebenden Rand gebildet ist.

Aus der US 2002/081109 A1 ist eine Vorrichtung zum Erwärmen von strömenden Fluiden mit einem Fluidgehäuse bekannt, das kassettenartig aus einem starren Kunststoffmaterial besteht. Zwischen einem stirnseitigen Einlass und einem stirnseitigen Auslass erstreckt sich ein mäanderförmiger Fluidkanal. Das Fluidgehäuse weist zwar Flachseiten auf. Es fehlen jedoch gänzlich Abdeckfolien, die Wandungen des Fluidkanals bilden.

Aus der US 2002/181948 A1 ist eine Vorrichtung zum Erwärmen von strömenden Fluiden bekannt, die ein einstückiges Fluidgehäuse aus einem starren Material vorsieht. Abdeckfolien zur Bildung von Fluidkanalwänden sind nicht offenbart.

Aus der US 7 316 666 B1 ist eine Vorrichtung zum Erwärmen von strömenden Fluiden bekannt, die ein Fluidgehäuse enthaltend einen mäanderförmigen Fluidkanal aufweist. Gegenüberliegende Wandungen des Fluidgehäuses werden durch Abdeckfolien gebildet. Zur Befestigung der Abdeckfolien bzw. Bildung des Fluidgehäuses sind Streben vorgesehen sowie eine Versteifungsplatte. Die Streben und der Versteifungsplatte sind randseitig zwischen den beiden Abdeckfolien angeordnet und ermöglichen die Stabilität des Fluidgehäuses.

Aus der US 2004/0026068 A1 ist eine Vorrichtung zum Erwärmen von strömenden Fluiden bekannt. Diese Vorrichtung weist ein Fluidgehäuse mit einem mäanderförmigen Fluidkanal auf, wobei Wandungen des Fluidgehäuses durch Abdeckfolien gebildet sind. Allerdings erfolgt eine Stabilisierung des Fluidgehäuses ausschließlich in einem Randbereich.

Aus der US 2004/0190885 A1 ist eine Vorrichtung zum Erwärmen von strömenden Fluiden mit einem Fluidgehäuse bekannt. Innerhalb des Fluidgehäuses ist ein mäanderförmiger Fluidkanal ausgebildet, der eine Anzahl von bogenförmigen Fluidkanalabschnitten und eine Anzahl von geraden Fluidkanalabschnitten aufweist. Zwischen benachbarten geraden Fluidkanalabschnitten erstreckt sich eine Stabilisierungszunge. Begrenzungskanten bzw. Wandungen benachbarter gerader Fluidkanalabschnitte verlaufen parallel zueinander.

Aufgabe der vorliegenden Erfindung ist es daher, eine Vorrichtung zum Erwärmen von strömenden Fluiden derart weiterzubilden, dass auf einfache Weise ein flaches und platzsparendes Fluidgehäuse geschaffen wird, an dem außenseitig eine Heizeinrichtung ansetzbar ist, so dass eine effektive Wärmeübertragung auf das strömende Fluid erzielt wird.

Zur Lösung dieser Aufgabe weist die Erfindung die Merkmale des Patentanspruches 1 auf.

Der besondere Vorteil der Erfindung besteht darin, dass ein stabiles und flaches Fluidgehäuse geschaffen wird, das einfach handhabbar und bezogen auf den Fluidkanalquerschnitt und der Fluidkanallänge einen relativ geringen Bauraum erfordert. Das erfindungsgemäße Fluidgehäuse weist einen kompakten Aufbau auf. Die Platzersparnis ergibt sich insbesondere dadurch, dass ein Flachrahmen einer Kontur des Fluidkanals folgend ausgebildet ist. Dadurch, dass Kanten des Flachrahmens zur Begrenzung des Fluidkanals an einer Schmalseite dienen, braucht die Abdeckfolie lediglich auf gegenüberliegenden Flachseiten des Flachrahmens befestigt sein.

Nach der Erfindung ist der Fluidkanal mäanderförmig ausgebildet, wobei der Flachrahmen eine Anzahl von geraden Fluidkanalabschnitten und eine Anzahl von bogenförmigen Fluidkanalabschnitten aufweist, die sich zwischen dem Einlass und dem Auslass des Fluidkanals erstrecken. Zwei benachbarte gerade Fluidkanalabschnitte sind durch eine Stabilisierungszunge begrenzt, wobei eine Spitze der Stabilisierungszunge eine Wandung in Strömungsrichtung zwischen zwei geraden Fluidkanalabschnitten angeordneten bogenförmigen Fluidkanal bildet. Hierdurch wird platzsparend eine Stabilisierung des Fluidgehäuses im Inneren desselben geschaffen. Ein Außenrand des Fluidgehäuses kann relativ schmal ausgebildet sein. Nach der Erfindung sind die Begrenzungskanten der bogenförmigen Fluidkanalabschnitte derart ausgebildet, dass sich der Fluidkanal zumindest in einem geraden Fluidkanalabschnitt in Strömungsrichtung verjüngt. Auf diese Weise wird das Abführen von unerwünschten Luftbläschen innerhalb des Fluidkanals begünstigt.

Nach einer bevorzugten Ausführungsform der Erfindung weist die Stabilisierungszunge eine mindestens doppelt so große Breite auf wie ein in Strömungsrichtung außenseitig angeordneter bogenförmiger Fluidkanalabschnitt, der zugleich auch den Außenrand des Fluidgehäuses bildet. Zwischen dem Einlass und dem Auslass werden in einem durch die Anzahl der bogenförmigen Fluidkanalabschnitte vorgegebenen vorzugsweise gleichen Abstand mehrere Stabilisierungszungen gebildet, die das Fluidgehäuse von innen her stabilisieren. Insbesondere bei rechteckförmig ausgebildeten Fluidgehäusen mit einer Vielzahl von bogenförmigen Fluidkanalabschnitten ergibt sich somit eine gute Stabilität des Fluidgehäuses, ohne dass die Gefahr besteht, dass es bei unsachgemäßer Behandlung abknicken kann.

Nach einer Weiterbildung der Erfindung weist der Fluidkanal eine flächige Form auf, da gegenüberliegende breite Wandungen durch die Abdeckfolie und gegenüberliegende schmale Wandungen durch Begrenzungskanten des Flachrahmens gebildet werden. Durch die flache Ausgestaltung des Fluidkanals kann eine Heizeinrichtung flächig unmittelbar auf die vorzugsweise ebene Abdeckfolie des Fluidkanals aufgebracht werden, so dass ein guter Wärmeeintrag gewährleistet ist.

Nach einer Weiterbildung der Erfindung verläuft eine obere Wandung eines dem Einlass zugewandten geraden Fluidkanalabschnittes in Verlängerung einer durch den Einlass vorgegebenen Strömungsrichtung des Fluids. Die obere Wandung, die als schmale Begrenzungskante ausgebildet ist, verläuft somit kollinear zu dem Einlass. Dadurch, dass die obere Wandung in der Montageposition kontinuierlich bezogen auf eine horizontale Richtung ansteigt, können vorhandene Gasbläschen einfach in Strömungsrichtung abgeführt werden.

Nach einer Weiterbildung der Erfindung sind an einer äußeren Seite von dem bogenförmigen Fluidkanalabschnitt eine Eingangswandung und/oder eine Ausgangswandung vorgesehen, so dass der bogenförmige Fluidkanalabschnitt den Fluidkanal entsprechend in einem Öffnungswinkelbereich von 40° bis 50° umlenkt. Vorteilhaft ermöglicht dieser Umlenkungsgrad eine optimale Strömungsführung.

Nach einer Weiterbildung der Erfindung ist eine erste Abdeckfolie mit einer ersten Flachseite des Flachrahmens und eine zweite Abdeckfolie mit einer zweiten Flachseite des Flachrahmens stoffschlüssig verbunden. Die erste und zweite Abdeckfolie erstrecken sich jeweils eben auf den jeweiligen Flachseiten und ermöglichen eine Flächenanlage der Heizeinrichtung an denselben.

Nach einer Weiterbildung der Erfindung weist das flächige Fluidgehäuse eine konstante Dicke auf, die durch den Abstand der gegenüberliegenden ersten Abdeckfolie und zweiten Abdeckfolie bzw. einer Dicke des Flachrahmens vorgegeben wird. Vorteilhaft weist der Fluidkanal eine im Querschnitt rechteckige Kontur auf, die eine vergrößerte Wärmeübertragungsfläche zu der Heizeinrichtung gewährleistet.

Nach einer Weiterbildung der Erfindung weist mindestens eine Stabilisierungszunge eine Lochung auf zur Befestigung der unmittelbar an beiden Seiten des Flachrahmens bzw. der Abdeckfolien anliegenden Heizeinrichtung. Die Befestigung der Heizeinrichtung kann somit platzsparend in einem Innenbereich des Flachrahmens erfolgen.

Weitere Vorteile der Erfindung ergeben sich aus den weiteren Unteransprüchen.

Ein Ausführungsbeispiel der Erfindung wird nachfolgend anhand der Zeichnungen näher erläutert.

Es zeigen:
- Figur 1: eine Draufsicht auf ein flächiges Fluidgehäuse,
- Figur 2: eine Seitenansicht des Fluidgehäuses,
- Figur 3: eine Draufsicht auf ein flächiges Fluidgehäuse mit einem Fluidkanalverlauf nach einer alternativen Ausführungsform und
- Figur 4: eine Seitenansicht eines schraubenförmigen Fluidgehäuses.

Eine erfindungsgemäße Vorrichtung zum Erwärmen von strömenden Fluiden wird vorzugsweise für intravenöse Fluide (VI-Fluide) eingesetzt. Bspw. dient die erfindungsgemäße Vorrichtung zum Erwärmen IV-Fluid, insbesondere von strömendem Blut oder einer Infusionsflüssigkeit, wobei die Infusionsflüssigkeit von einem Flüssigkeitsspeicher zu einem Patienten fließt. Die erfindungsgemäße Vorrichtung weist ein flächiges Fluidgehäuse 1 auf, das im Wesentlichen durch einen einstückigen Flachrahmen 2 und einer Abdeckfolie 3 gebildet ist.

Der Fluidrahmen 2 weist eine einem Fluidkanal 4 folgende Form auf. Im vorliegenden Ausführungsbeispiel ist der Fluidkanal 4 mäanderförmig ausgebildet, wobei er sich bogenförmig zwischen einem unteren Einlass 5 und einem oberen Auslass 6 erstreckt. Das Fluidgehäuse 1 ist vorzugsweise vertikal in einer nicht dargestellten Halterung positioniert. Im vorliegenden Ausführungsbeispiel weist der Flachrahmen 2 bzw. der Fluidkanal 4 einen ersten bogenförmigen Fluidkanalabschnitt 7, einen in Strömungsrichtung S vor dem ersten bogenförmigen Fluidkanalabschnitt 7 verlaufenden zweiten bogenförmigen Fluidkanalabschnitt 7' sowie einen weiteren stromabwärts zu dem zweiten bogenförmigen Fluidkanalabschnitt 7' angeordneten dritten bogenförmigen Fluidkanalabschnitt 7". Der erste bogenförmige Fluidkanal 7 ist über einen ersten geradlinigen Fluidkanalabschnitt 12 mit dem Einlass 5 und über einen zweiten geraden Fluidkanalabschnitt 12" mit einem zweiten bogenförmigen Fluidkanalabschnitt 7' verbunden, der zweite bogenförmige Fluidkanalabschnitt 7' ist über einen dritten geraden Fluidkanalabschnitt 12" mit einem dritten bogenförmigen Fluidkanalabschnitt 7' und der dritte bogenförmige Fluidkanalabschnitt 7" ist über einen dritten geraden Fluidkanalabschnitt 12" mit dem Auslass 6 verbunden. Die bogenförmigen Fluidkanalabschnitte 7, 7', 7" weisen jeweils eine innere Wandung 8 und eine äußere Wandung 9 auf. Die innere Wandung 8 und die äußere Wandung 9 der bogenförmigen Fluidkanalabschnitte 7, 7', 7" verlaufen jeweils über die gesamte Länge bogenförmig bzw. U-förmig. Sie weisen somit einen kontinuierlichen Verlauf auf.

Die geraden Fluidkanalabschnitte 12, 12', 12" und die, 12‴ bogenförmigen Fluidkanalabschnitte 7, 7', 7" verlaufen in Strömungsrichtung alternierend. Da die bogenförmigen Fluidkanalabschnitte 7, 7', 7" sich in einem Bogen von etwa 180° erstrecken, liegen die geraden Fluidkanalabschnitte 12, 12', 12", 12‴ nebeneinander, wobei sie durch eine Stabilisierungszunge 10 begrenzt sind. Eine Spitze dieser Stabilisierungszunge wird durch die innere Wandung 8 der bogenförmigen Fluidkanalabschnitte 7, 7', 7" gebildet.

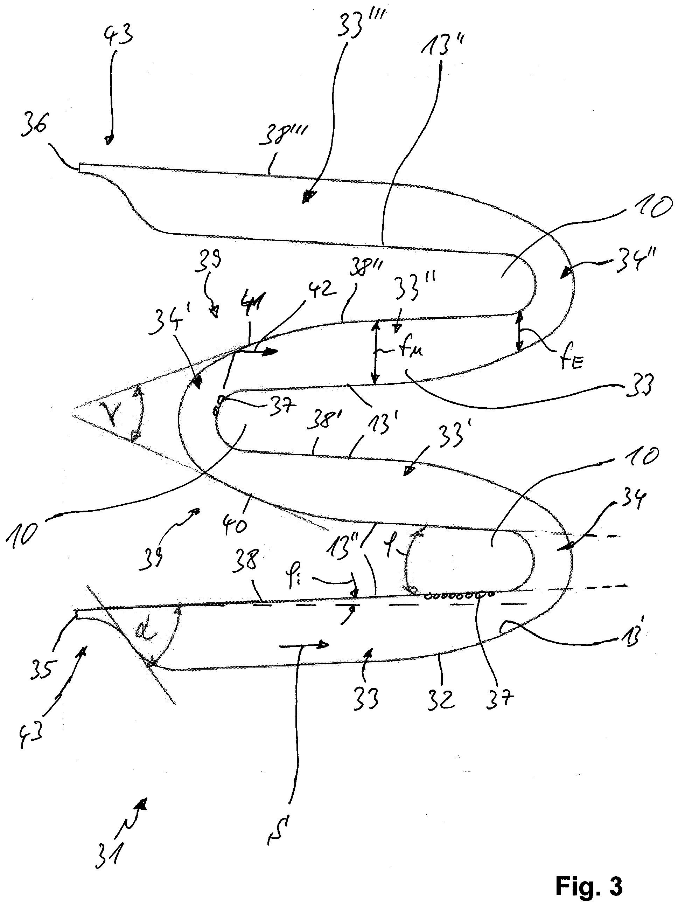

Die geraden Fluidkanalabschnitte 12, 12', 12", 12‴ verlaufen im Vergleich zu den bogenförmigen Fluidkanalabschnitten 7, 7', 7" geradlinig. Wie aus Figur 3 ersichtlich ist, können die geraden Fluidkanalabschnitte 33', 33", 33‴ teilweise auch nichtgerade Wandungen aufweisen, insbesondere im Übergang zu den bogenförmigen Fluidkanalabschnitten'34, 34', 34".

An Enden des Fluidkanals 4 verläuft der Fluidkanal 4 bzw. der Flachrahmen 2 konusförmig zu dem Einlass 5 bzw. Auslass 6 zusammen. Die Stabilisierungszunge 10 weist eine Breite bₛ auf, die größer ist als eine doppelte Breite b_{A} der äußeren Wandung 9 des bogenförmigen Fluidkanalabschnittes 7, 7', 7".

Im vorliegenden Ausführungsbeispiel sind drei Stabilisierungszungen 10 vorgesehen, die in einem Abstand zueinander und in einem inneren Bereich des flächigen Fluidgehäuses angeordnet sind. Eine Formstabilität des Fluidgehäuses 1 wird somit durch innere Abschnitte des Flachrahmens gebildet. Ein Außenrand des Fluidgehäuses 1 bzw. des Flachrahmens 2 wird im Wesentlichen durch die relativ schmale äußere Wandung 9 des bogenförmigen Fluidkanalabschnittes 7, 7', 7" gebildet.

Die erste Abdeckfolie 3' ist stoffschlüssig mit einer ersten Flachseite 2' des Flachrahmens 2 verbunden. Die zweite Abdeckfolie 3" ist stoffschlüssig mit einer zweiten Flachseite 2" des Flachrahmens 2 verbunden. Die erste Abdeckfolie 3' bzw. die zweite Abdeckfolie 3" sind jeweils durch Verschweißen oder Kleben oder durch Anspritzen an der ersten Flachseite 2" bzw. der zweiten Flachseite 2" befestigt.

Der Fluidkanal 4 ist im Querschnitt rechteckförmig ausgebildet, wobei er gegenüberliegende breite Wandungen aufweist, die durch die erste Abdeckfolie 3' bzw. die zweite Abdeckfolie 3" gebildet ist. Ferner ist der Fluidkanal 4 durch gegenüberliegende schmale Wandungen begrenzt, die jeweils durch eine erste und zweite innere Begrenzungskante 13', 13" des Flachrahmens 2 gebildet ist. Die erste Begrenzungskante 13' und die zweite Begrenzungskante 13" des Fluidrahmens 2 verlaufen abwechselnd als Bestandteil der bogenförmigen Fluidkanalabschnitte 7, 7', 7" und des geraden Fluidkanalabschnittes 12, 12', 12", 12‴ in Strömungsrichtung S. Im vorliegenden Ausführungsbeispiel ist der Abstand der gegenüberliegenden Begrenzungskanten 13', 13" des geraden Fluidkanalabschnittes 12, 12', 12", 12‴ und des bogenförmigen Fluidkanalbschnittes 7, 7', 7" zueinander mindestens 4-mal so groß wie der Abstand der breiten Wandungen des Fluidkanals 4 zueinander. Auf diese Weise ist der Fluidkanal 4 relativ flach ausgebildet, wobei mittels der Stabilisierungszunge 10 eine relativ große Anschlag- oder Ansatzfläche gebildet wird für eine nicht dargestellte elektrische Heizeinrichtung, die mit einer Heizplatte jeweils flächig an der ersten Abdeckfolie 3' und an der ersten Flachseite 2' bzw. der zweiten Abdeckfolie 3" und der zweiten Flachseite 2" anliegt.

Die Heizeinrichtung kann bspw. als induktive Heizeinrichtung oder als eine magnetoresistive Heizungseinrichtung oder als eine Widerstandsheizung oder als eine durch Infrarotlichtquellen oder Laserlichtquellen betriebene Heizeinrichtung ausgebildet sein.

Das Fluidgehäuse 1 weist im Bereich des ersten bogenförmigen Fluidkanalabschnittes 7, des zweiten bogenförmigen Fluidkanalabschnittes 7' und des dritten bogenförmigen Fluidkanalabschnittes 7" eine konstante Dicke d auf. In diesem Bereich verlaufen die erste Flachseite 2' und die zweite Flachseite 2" des Flachrahmens 2 eben. Die erste Flachseite 2' und die zweite Flachseite 2" laufen symmetrisch zu einer Längsmittelebene des Fluidgehäuses 1. Lediglich im Bereich des Einlasses 5 und des Auslasses 6 verlaufen die erste Flachseite 2' und die zweite Flachseite 2" jeweils konusförmig zusammen, wobei die Dicke d zwischen der ersten Flachseite 2' und der zweiten Flachseite 2" in Richtung des Fluideinlasses 5 bzw. Fluidauslasses 6 zu einer Dicke d_{E} zunimmt. Der Querschnitt des Fluidkanals 4 entspricht im Wesentlichen dem Querschnitt einer Rohrform des Einlasses 5 bzw. Auslasses 6, so dass innerhalb des Fluidgehäuses 1 keine Druckschwankungen entstehen.

Der gerade Fluidkanalabschnitt 12, 12', 12", 12‴ ist nur annähernd gerade ausgebildet. Der gerade Fluidkanalabschnitt 12, 12', 12"kann in Strömungsrichtung S leicht verjüngend ausgebildet sein, damit eventuell entstehende Luftblasen innerhalb des vertikal angeordneten Fluidgehäuses 1 einfacher nach oben in Richtung des Fluidauslasses 6 abgeführt werden können. Zur Bildung der Verjüngung verlaufen die Begrenzungskante 13' und/oder der Begrenzungskante 13" der geraden Fluidkanalabschnitte 12, 12', 12", 12‴ unter einem Verjüngungswinkel ϕⱼ von mindestens 3° bezüglich einer Achse A des rohrförmigen Fluideinlasses 5 bzw. einer zu derselben parallelen Mittelachse der geraden Fluidkanalabschnitte 12, 12', 12". Da die Begrenzungskante 12" eine Wandung der Stabilisierungszunge 10 bildet, verläuft die Stabilisierungszunge 10 zumindest an einer Seite verjüngend in Richtung zu einer Spitze derselben. Da beide Begrenzungskanten 13', 13", eine Verjüngung aufweisen, ergibt sich somit eine gesamte Verjüngung mit einem Winkel von 3°. In Abhängigkeit von Randparametern kann der Verjüngungswinkel ϕ auch 4° bis 5° betragen, so dass die Gesamtverjüngung 8° bis 10° beträgt. Der Verjüngungswinkel ϕ verläuft in einer Erstreckungsebene des Flachrahmens 2 bzw. der Flachseiten 2', 2".

Wie aus den Figuren ersichtlich ist, ermöglichen die bogenförmigen Fluidkanalabschnitte 7, 7', 7" eine Umlenkung der Fluidströmung um 180°, so dass das Fluidgehäuse 1 bei ungerader Anzahl der bogenförmigen Fluidkanalabschnitte 7, 7', 7" so ausgebildet ist, dass der Fluideinlass 5 und der Fluidauslass 6 auf der gleichen Seite und vorzugsweise in der gleichen Querebene zu dem Fluidgehäuse 1 angeordnet sind. Eine Anbindung an entsprechende Schläuche kann somit einfach vorgenommen werden.

Im vorliegenden Ausführungsbeispiel weist der Flachrahmen 2 in einem inneren Bereich Lochungen 14 auf zur Befestigung des Fluidgehäuses 1 an der benachbarten Heizeinrichtung. Die Lochungen 14 sind in mindestens zwei Stabilisierungszungen 10 vorgesehen, so dass eine definierte Position des Fluidgehäuses 1 in der Heizeinrichtung gewährleistet ist.

Im vorliegenden Ausführungsbeispiel sind in einem zum Fluideinlass 5 nahen Bereich sowie in einem zum Fluidauslass 6 nahen Bereich sowie in einem mittleren Bereich des Fluidkanals 4 Sensoren 15 angeordnet, die jeweils über eine Leitung 16 zu einer Kontaktierungsleiste 17 des Fluidrahmens 2 führen. Die Sensoren 15 können als Temperatursensoren und/oder als Fließgeschwindigkeitssensoren und/oder als Feuchtesensoren ausgebildet sein. Die Sensoren 15 und die Leitungen 16 können auf der ersten Abdeckfolie 3' aufgelegt sein bzw. bspw. durch Kleben mit derselben verbunden sein.

Alternativ können die Sensoren 15 und die Leitungen 16 auch unter Anlage an der ersten Flachseite 2" bspw. stoffschlüssig befestigt sein, wobei bei dieser Variante der Erfindung die Abdeckfolie 3' erst nach Festlegen der Temperatursensoren 15 und der Leitungen 16 auf die erste Flachseite 2' aufgebracht wird. Die Leitungen 16 sind bei dieser Variante steif oder starr ausgebildet.

Der einstückige Flachrahmen 2 kann durch Stanzen oder durch Spritzgießen hergestellt sein. Er besteht aus einem Kunststoffmaterial. Die Abdeckfolie 3', 3" wird zur Herstellung des Fluidgehäuses 1 jeweils flächig auf die entsprechenden Flachseiten 2', 2" gelegt und mittels Wärme und Druck von einer Folienbahn entfernt und zugleich haftend an dem Flachrahmen 2 angebracht.

Vorzugsweise ist der Einlass 5 und der Auslass 6 als ein Luer-Lock-Verbindungsanschluss ausgebildet, sodass auf einfache Weise Schläuche mit dem Flachrahmen 2 verbunden werden können.

Nach einer weiteren Ausführungsform der Erfindung kann der Sensor eine Spektrometereinheit und eine Sensorleitung aufweisen, wobei die Sensorleitung zu dem Fluidgehäuse 1 hinführbar ist. Die Sensorleitung ist über ein erstes Ende mit der Spektrometereinheit verbunden. Ein zweites Ende der Sensorleitung ist so ausgebildet, dass eine freie Stirnfläche der Sensorleitung am zweiten Ende in einer solchen vorgegebenen Relativposition zu dem Fluidgehäuse 1 angeordnet ist, dass eine Messung von Eigenschaften des Fluids ermöglicht wird. Das zweite Ende der Sensorleitung kann mit einem Halteelement versehen sein, sodass die Sensorleitung lösbar mit dem Fluidgehäuse 1 verbindbar ist. Hierbei kann das Halteelement als ein das zweite Ende verbreiternder Kopf ausgebildet sein, sodass das zweite Ende mit den Stirnflächen flächig unmittelbar an der Außenseite der Abdeckfolie 3' anliegt. Alternativ kann das zweite Ende der Sensorleitung auch an dem Flachrahmen 2 befestigt sein, wobei durch Ausgestaltung des zweiten Endes sichergestellt ist, dass die freie Stirnfläche des zweiten Endes unmittelbar an der Außenseite der Abdeckfolie 3' oder in einem definierten Abstand zu derselben positioniert ist.

Vorzugsweise ist die Spektrometereinheit als eine optische Reflexionsspektrometereinheit ausgebildet. Die Sensorleitung weist mindestens zwei Lichtleiter auf. Ein erster Lichtleiter der Sensorleitung dient zur Zuführung von Licht zu dem Fluidgehäuse 1 bzw. zu dem Fluid. Ein zweiter Lichtleiter der Sensorleitung dient zur Zurückleitung des mittels des ersten Lichtleiters zu dem Fluid zugeführten und an demselben reflektierten Lichtes zu der optischen Reflexionsspektrometereinheit. In einer mit der Reflexionsspektrometereinheit verbundenen Auswertereinheit kann auf Basis eines Algorithmus die Eigenschaft des Fluids ermittelt werden. Wenn es sich bei dem Fluid um ein Blut handelt, kann hierdurch bspw. der Hämoglobinwert (HB-Wert) ermittelt werden.

Alternativ kann statt des Reflexionsspektrums auch das Absorptionsspektrum des Fluids ermittelt werden, woraus die entsprechenden Eigenschaften des Fluids bestimmt werden.

Ein Vorteil dieser Ausführungsform besteht darin, dass der Sensor (Messeinrichtung) weiterbenutzt werden kann, auch wenn das Fluidgehäuse 1 bspw. für einen neuen Patienten ersetzt wird. Damit kann das Fluidgehäuse als kostengünstig hergestelltes Wegwerfteil (Kassette) dienen, während der Sensor und/oder die Heizeinrichtung stets wiederverwendbar sind.

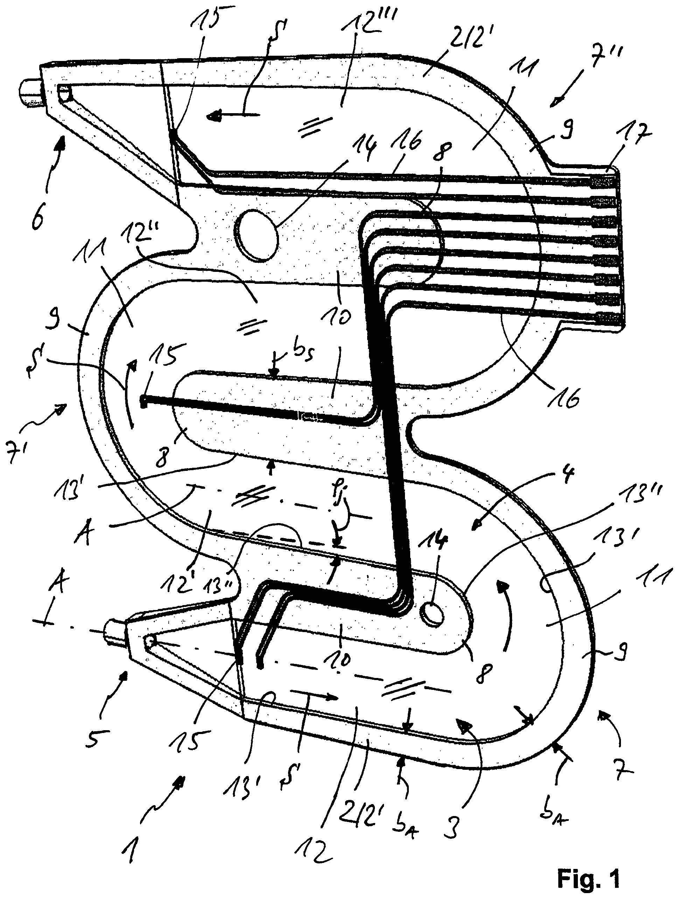

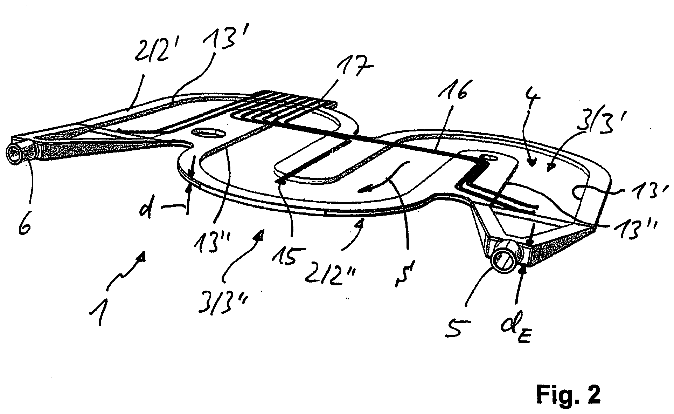

Nach einer weiteren Ausführungsform der Erfindung gemäß Figur 3 ist ein flächiges Fluidgehäuse 31 vorgesehen, das - wie im vorherigen Ausführungsbeispiel - einen einstückigen Flachrahmen 32 sowie eine Abdeckfolie aufweist. Im Unterschied zu dem vorgenannten ersten Ausführungsbeispiel gemäß den Figuren 1 und 2 ist der Fluidkanalverlauf ein anderer. Die Temperatursensoren sind zur besseren Darstellung in der Figur 3 nicht eingezeichnet. Es versteht sich, dass diese in dem Fluidgehäuse 31 integriert sein können. Auch liegen an der einen und/oder anderen Flachseite des Fluidgehäuses 31 vorzugsweise ausschließlich im Bereich der Abdeckfolie Heizelemente an zum gesteuerten Temperieren des innerhalb des Fluidgehäuses 31 strömenden Fluids.

Das Fluidgehäuse 31 wird in der Gebrauchsstellung senkrecht gehaltert, wobei von einem unteren Einlass 35 das Fluid in Strömungsrichtung S in Richtung eines oberen Auslasses 36 strömt.

Der Flachrahmen bzw. der Fluidkanal 32 weist eine Anzahl von geraden Fluidkanalabschnitten 33, 33', 33", 33‴ und einer Anzahl von bogenförmigen Fluidkanalabschnitten 34, 34', 34" auf, die sich in Strömungsrichtung S abwechseln und einen mäanderförmigen Verlauf ermöglichen. Die geraden Fluidkanalabschnitte 33 und die bogenförmigen Fluidkanalabschnitte 34 werden durch gegenüberliegende Begrenzungskanten 13', 13" sowie auf dem Flachrahmen 32 aufgebrachte Abdeckfolien 3, 3', 3" begrenzt. Der Fluidkanal 32 ist relativ flach ausgebildet, weist eine relativ geringe Dicke d auf, so dass eine effektive Wärmeeinleitung von den an den Flachseiten angeordneten Heizelementen gewährleistet ist.

Damit unerwünschte Gasblasen 37, die in vertikaler Richtung gesehen an einer oberen Wandung 38, 38', 38" des geraden Fluidkanalabschnittes 33, 33', 33", 33‴ anhaften bzw. anliegen, weist die Stabilisierungszunge 10 in Richtung des bogenförmigen Fluidkanalabschnittes 34 eine sich unter einem Verjüngungswinkel ϕⁱ verjüngende Begrenzungskante 13'und/oder Begrenzungskante 13" auf. Wie besser aus Figur 3 zu ersehen ist, schließen die beiden Begrenzungskanten 13', 13" der Stabilisierungszunge 10 einen Öffnungswinkel ϕ im Bereich von 4° bis 8° ein. Durch diese Steigung des Fluidkanals 32 bezogen auf eine horizontale Ebene im Bereich des geraden Fluidkanalabschnittes 33, 33', 33" wird eine Wanderung der Gasbläschen 37 in Strömungsrichtung S hervorgerufen. Dasselbe gilt auch für eine obere Wandung 38‴ des geraden Fluidkanalabschnittes 33‴.

In dem Bereich des bogenförmigen Fluidkanalabschnittes 34, 34', 34" weist der Fluidkanal 32 an einer äußeren Seite 39 eine Eingangswandung 40 und eine Ausgangswandung 41 auf. Diese Schrägwandungen 40, 41 ermöglichen ein leichteres Abdriften der an derselben anhaftenden Gasbläschen 37 in Strömungsrichtung S. Insbesondere die Ausgestaltung in der Ausgangswandung 41 ist für die Abführung der Gasbläschen 37 in Strömungsrichtung S förderlich, da die an der oberen Wandung 38 des zweiten geraden Fluidkanalabschnittes 33' anhaftenden Gasbläschen 37 nach Eintritt in den zweiten bogenförmigen Fluidkanalabschnitt 34' unmittelbar auf die bogenförmige Außenwandung 41 des zweiten bogenförmigen Fluidkanalabschnittes 34' treffen und dann an die obere Wandung 38" des dritten geraden Fluidkanalabschnittes 33" weitergeleitet wird, siehe Pfeil 42.

Die Eingangswandung 40 und die Ausgangswandung 41 des bogenförmigen Fluidkanalabschnittes 34, 34', 34" bilden einen Öffnungswinkelbereich γ im Bereich von 40° bis 50°. Vorzugsweise beträgt der Öffnungswinkel γ 45°.

Die obere Wandung 38 des ersten geraden Fluidkanalabschnittes 33, der dem Einlass 5 zugewandt ist, erstreckt sich in Strömungsrichtung unter einem Winkel ϕ im Bereich zwischen 2° und 4°. Vorzugsweise weist die schräg ansteigende obere Wandung 38 des ersten geraden Fluidkanalabschnittes 33 zu der Horizontalen einen Winkel ϕ von 3° auf. Die obere Wandung 38 des ersten geraden Fluidkanalabschnittes 33, die durch die Begrenzungskante 13', 13" gebildet ist, verläuft in geradliniger Verlängerung eines durch den Einlass 5 vorgegebenen Strömungsrichtung S des Fluids. Die obere Wandung 38 des ersten Fluidkanalabschnittes 33 verläuft somit im Wesentlichen geradlinig bzw. leicht gewölbt bis zu dem Einlass 5. Der Verlauf der oberen Wandung 38 erfolgt im Wesentlichen ohne Kanten, d. h. stetig. Hierdurch ist gewährleistet, dass die Gasblasen 37 in Strömungsrichtung entlang der oberen Wandung 38 des ersten Fluidabschnittes 33 entlang wandern können in Richtung des ersten bogenförmigen Fluidkanalabschnittes 34, von wo sie aus zu der oberen Wandung 38 des zweiten Fluidkanalabschnittes 33' gelangen und so weiter, bis sie aus dem Auslass 36 den Fluidkanal 32 verlassen.

Die obere Wandung 38" des ersten Fluidkanalabschnittes 33 kann kollinear zu dem Einlass 35 verlaufen.

Vorzugsweise ist der geradlinige Fluidkanalabschnitt 33, 33', 33", 33‴ in Richtung zu dem bogenförmigen Fluidkanalabschnitt 34, 34', 34" und/oder zu dem Einlass 35 und/oder zu dem Auslass 36 verjüngend ausgebildet.

In Richtung eines zu dem Einlass 35 und dem Auslass 36 zugewandten Ende 43 des Fluidkanals 32 verläuft derselbe verjüngend unter einem Verjüngungswinkelbereich ϕ von 50° bis 70°. Beispielsweise kann der Verjüngungswinkel α 60° betragen, siehe Figur 3. Das dem Einlass 35 und dem Auslass 36 zugewandte Ende 43 weist somit eine pelikanschnabelförmige Form auf.

Es ist ersichtlich, dass die geradlinigen Fluidkanalabschnitte 33, 33', 33", 33‴ bauchig ausgebildet sind, wobei sie zu den jeweiligen Enden eine kleinere Breite f_{E} aufweisen. Eine Breite f_{M} im mittleren Bereich der geraden Fluidkanalabschnitte 33 ist somit größer als eine Breite f_{E} im Endbereich derselben.

Nach einer weiteren alternativen Ausführungsform der Erfindung muss der Fluidkanal nicht in einer Ebene verlaufen, wie bei den oben genannten Ausführungsbeispielen. Er kann auch schraubenförmig (helixförmig) um eine Achse (Mittelachse) verlaufend angeordnet sein. Verläuft die Achse in vertikaler Richtung, verlaufen die Flachseiten des Fluidkanals koaxial bzw. die Flachseiten des Fluidkanals verlaufen in Umfangsrichtung. Der so gebildete spulenförmige Fluidkanal weist somit Flachwindungen auf, deren gegenüberliegende schmale Begrenzungskanten eine zu der Achse parallele Ebene aufspannen.

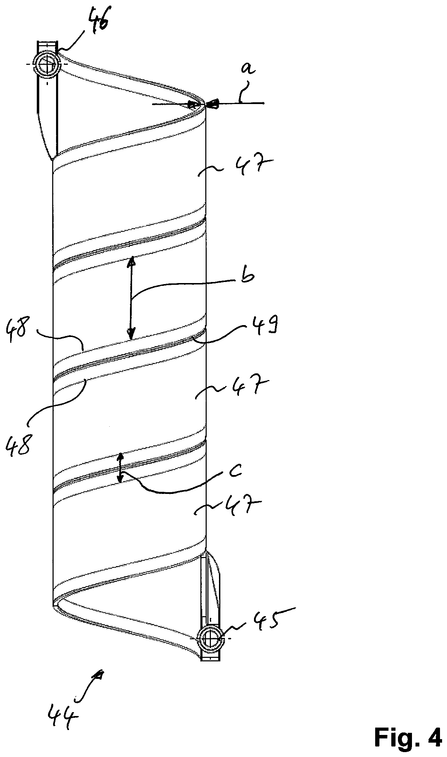

In Figur 4 ist schematisch der Verlauf eines schraubenförmigen Fluidgehäuses 44 dargestellt. An einer unteren Seite des Fluidgehäuses 44 ist ein Fluideinlass 45 und an einer oberen Seite desselben ein Fluidauslass 46 angeordnet, die jeweils rohrförmig ausgebildet sind. Zwischen dem Fluideinlass 45 und dem Fluidauslass 46 erstreckt sich ein schraubenförmiger Fluidkanal 47 als Flachkanal, der vorzugsweise eine konstante Dicke a und eine konstante Breite b aufweist. Schmalseiten 48 benachbarter Abschnitte des Fluidkanals 47 sind in einem Abstand c zueinander angeordnet. Es bildet sich ein mäanderförmiger Kanalverlauf in axialer Richtung des Fluidgehäuses 44 aus. Der schraubenförmige Fluidkanal 47 weist somit Flachseiten auf, die senkrecht zu der Achse (Mittelachse) des schraubenförmigen Fluidgehäuses 44 verlaufen. Die Schmalseiten 48 sind aus einem starren Material gebildet, wobei sich zwischen zueinander gekehrten Schmalseiten 48 von in Achsrichtung benachbarten Abschnitten des Fluidkana147 eine schraubenförmige Grenzlinie 49 bildet. Die Schmalseiten 48 sind vorzugsweise einstückig ausgebildet.

Zur Bildung des schraubenförmigen Fluidgehäuses 44 werden die Schmalseiten 48 bzw. Begrenzungskanten des Fluidkanals 47 aus einen starren Material, vorzugsweise aus einem Kunststoffmaterial, gespritzt. Sie bilden ein starres Gerüst oder Gerippe. Zur Bildung von Flachseiten des Fluidkanals 47 werden die Schmalseiten 48 auf einer achsenzugewandten Seite und einer achsenabgewandten Seite mit einer vorzugsweise transparenten Abdeckfolie stoffschlüssig verbunden. Das Fluidgehäuse 44 ist als ein Hohlzylinder ausgebildet, wobei eine Innenseite und eine Außenseite des Hohlzylinders durch die Abdeckfolie gebildet ist.

Nach einer zweiten Ausführungsform des schraubenförmigen Fluidgehäuses werden zwei vorzugsweise transparente Abdeckfolien in Schlauchform koaxial ineinander gesetzt, wobei die eine Abdeckfolie einen größeren Durchmesser aufweist als die andere. Anschließend wird der schraubenförmige Verlauf des Fluidkanals durch Verschweißen der einen Abdeckfolie mit der andern Abdeckfolie erzeugt. Die Schweißlinie verläuft spiralförmig und/oder schraubenförmig und stimmt mit dem Verlauf der Grenzlinie 49 des Fluidgehäuses 44 überein. An den gegenüberliegenden Stirnseiten der beiden Abdeckfolien erfolgt ebenfalls ein Verschweißen derselben zum dichten Abschließen des Fluidgehäuses an seinen Stirnseiten, wobei vorzugsweise der Fluideinlass und der Fluidauslass mit angeschweißt wird.

Das Fluidgehäuse wird somit durch zwei miteinander verschweißten Abdeckfolien gebildet, die nicht starr ausgebildet sind. Vorteilhaft vereinfacht sich hierdurch die Herstellung.

Die Abdeckfolie kann beispielsweise eine Dicke von 100µm bis 200µm aufweisen.

## Patentansprüche

1. Vorrichtung zum Erwärmen von strömenden Fluiden, insbesondere von intravenösen Fluiden, mit einem flächigen Fluidgehäuse (1) enthaltend einen Fluidkanal (4), durch den zwischen einem Einlass (5) und einem Auslass (6) das Fluid leitbar ist mit einer Abdeckfolie (3', 3") und mit einer außenseitig des Fluidgehäuses (1) angeordneten Heizeinrichtung zur Erwärmung des durch den Fluidkanal (4) strömenden Fluids, wobei der Fluidkanal (4) mäanderförmig ausgebildet ist, dass das Fluidgehäuse (1) einen Flachrahmen (2) mit einer eine Kontur des Fluidkanals (4) folgenden Form aufweist, dass der Flachrahmen (2) zu beiden Seiten einer Längsmittelebene desselben eine Flachseite (2', 2") aufweist und dass die beiden Flachseiten (2', 2") jeweils mit einer Abdeckfolie (3', 3") stoffschlüssig verbunden sind, so dass Wandungen des Fluidkanals (4) durch die zwei gegenüberliegenden Abdeckfolien (3', 3") und durch gegenüberliegende Begrenzungskanten (13', 13") des Flachrahmens (2) gebildet sind, dass der Flachrahmen (2) eine Anzahl von bogenförmigen Fluidkanalabschnitten (7, 7', 7") und eine Anzahl von geraden Fluidkanalabschnitten (12) aufweist, wobei sich zwischen Begrenzungskanten (13', 13") benachbarter gerader Fluidkanalabschnitte (12) eine Stabilisierungszunge (10) ausbildet, **dadurch gekennzeichnet, dass** die Stabilisierungszunge (10) in Richtung des bogenförmigen Fluidkanalabschnittes (34) eine sich unter einem Verjüngungswinkel (ϕᵢ) verjüngend verlaufende Begrenzungskante (13', 13") aufweist.

2. Vorrichtung nach Anspruch 1, **dadurch gekennzeichnet, dass** eine Wandung des Fluidkanals (4) durch die Abdeckfolie (3', 3") und eine Wandung des Fluidkanals (4) durch die Begrenzungskante (13', 13") des Flachrahmens (2) gebildet sind.

3. Vorrichtung nach Anspruch 1 oder 2, **dadurch gekennzeichnet, dass** die in Richtung des bogenförmigen Fluidkanalabschnittes (11, 34) verlaufenden beiden Begrenzungskanten (13', 13") der Stabilisierungszunge einen Öffnungswinkel (ϕ) im Bereich von 4° bis 8°, vorzugweise 6° aufspannen.

4. Vorrichtung nach einem der Ansprüche 1 bis 3, **dadurch gekennzeichnet, dass** der sich an den Einlass (35) anschließende gerade Fluidkanalabschnitt (33) des Fluidkanals (4) eine obere Wandung (38') aufweist, die geradlinig und in Verlängerung des Einlasses (35) verläuft.

5. Vorrichtung nach einem der Ansprüche 1 bis 4, **dadurch gekennzeichnet, dass** der bogenförmige Fluidkanalabschnitt (34) an einer äußeren Seite (39) desselben eine Eingangswandung (40) und eine Ausgangswandung (41) aufweist, die einen Öffnungswinkelbereich (γ) im Bereich von 40° bis 50°, vorzugsweise 45°, aufspannen.

6. Vorrichtung nach einem der Ansprüche 1 bis 5, **dadurch gekennzeichnet, dass** ein dem Einlass (5) und/oder dem Auslass (6) zugewandtes Ende (43) des geradlinigen Fluidkanalabschnittes (33, 33^{IV}) in Richtung des Einlasses (5) bzw. des Auslasses (6) unter einem Verjüngungswinkelbereich (ϕ) von 50° bis 70°, vorzugsweise 60°, verjüngend verläuft.

7. Vorrichtung nach einem der Ansprüche 1 bis 6, **dadurch gekennzeichnet, dass** eine erste Abdeckfolie (3') mit einer ersten Flachseite (2') und eine gegenüberliegende zweite Abdeckfolie (3") mit einer zweiten Flachseite (2") des Flachrahmens (2) stoffschlüssig verbunden ist.

8. Vorrichtung nach einem der Ansprüche 1 bis 7, **dadurch gekennzeichnet, dass** die Heizeinrichtung durch zwei Heizflächen gebildet ist, wobei eine erste Heizfläche flächig an der ersten Abdeckfolie (3') und eine zweite Heizfläche flächig an der zweiten Abdeckfolie (3") anliegt.

9. Vorrichtung nach einem der Ansprüche 1 bis 8, **dadurch gekennzeichnet, dass** im Bereich des Einlasses (5) und/oder im Bereich des Auslasses (6) ein Sensor (15), insbesondere ein Temperatursensor angeordnet ist und/oder eine Zuführungsleitung (16).

10. Vorrichtung nach einem der Ansprüche 1 bis 9, **dadurch gekennzeichnet, dass** mehrere Sensoren (15) an einer Außenseite der ersten Abdeckfolie (3') oder zwischen der ersten Abdeckfolie (3') und der ersten Flachseite (2') des Flachrahmens (2) in einer Ebene angeordnet sind.

11. Vorrichtung nach einem der Ansprüche 1 bis 10, **dadurch gekennzeichnet, dass** der Sensor eine Spektrometereinheit und eine Sensorleitung aufweist, wobei ein erstes Ende der Sensorleitung mit der Spektrometereinheit verbunden ist und wobei ein zweites Ende der Sensorleitung derart ausgebildet ist, dass eine Stirnfläche des zweiten Endes der Sensorleitung in einer vorgegebenen Relativposition unmittelbar an der Außenseite der Abdeckfolie (3') oder in einem definierten Abstand zu der Außenseite der Abdeckfolie (3') positioniert ist.

12. Vorrichtung nach Anspruch 11, **dadurch gekennzeichnet, dass** die Spektrometereinheit als eine optische Reflexionspektrometereinheit oder als eine optische Absorptionsspektrometereinheit ausgebildet ist und dass die Sensorleitung mindestens zwei Lichtleiter aufweist, wobei über einen ersten Lichtleiter Licht zu dem Fluid zugeführt und über einen zweiten Lichtleiter an dem Fluid reflektiertes Licht zu der Reflexionsspektrometereinheit bzw. Absorptionsspektrometereinheit zurückgeleitet wird, sodass auf Basis eines durch die Reflexionspektrometereinheit ermittelten Reflexionsspektrums oder eines auf Basis der durch die Absorptionsspektrometereinheit ermittelten Absorptionsspektrums des Fluids in einer Auswerteeinheit eine Eigenschaft des Fluids bestimmbar ist.

13. Vorrichtung nach einem der Ansprüche 1 bis 12, **dadurch gekennzeichnet, dass** sich das Fluidgehäuse (4) in einer Ebene erstreckt.

14. Vorrichtung nach einem der Ansprüche 1 bis 12, **dadurch gekennzeichnet, dass** das Fluidgehäuse (44) schraubenförmig verläuft mit einem schraubenförmigen Fluidkanal (47).

15. Vorrichtung nach Anspruch 14, **dadurch gekennzeichnet, dass** der schraubenförmige Fluidkanal (47) Flachseiten aufweist, die senkrecht zu einer Achse des schraubenförmigen Fluidgehäuses (44) verlaufen.

16. Vorrichtung nach Anspruch 14 oder 15, **dadurch gekennzeichnet**, das der schraubenförmige Fluidkanal (47) durch Aufkleben einer Abdeckfolie von einer achsennahen Seite und einer achsenfernen Seite aus auf ein starres spiralförmiges Gerüst herstellbar ist.

17. Vorrichtung nach einem der Ansprüche 14 bis 16, **dadurch gekennzeichnet**, das der schraubenförmige Fluidkanal durch Verschweißen zweier schlauchförmiger, koaxial ineinander gesetzter Abdeckfolien mit einer schraubenförmigen Schweißlinie herstellbar ist.

## Claims

1. A device for heating flowing fluids, in particular intravenous fluids, with a flat fluid housing (1) containing a fluid duct (4), through which the fluid can be conveyed between an inlet (5) and an outlet (6), with a covering film (3', 3") and with a heating means arranged on the outside of the fluid housing (1) for heating the fluid flowing through the fluid duct (4), the fluid duct (4) being serpentine in shape, the fluid housing (1) having a flat frame (2) with a shape following a contour of the fluid duct (4), the flat frame (2) having a flat side (2', 2") on either side of a longitudinal central plane thereof and the two flat sides (2', 2") being in each case materially bonded together with a covering film (3', 3"), such that walls of the fluid duct (4) are formed by the two opposing covering films (3', 3") and by opposing delimiting edges (13', 13") of the flat frame (2), the flat frame (2) having a number of arcuate fluid duct portions (7, 7', 7") and a number of straight fluid duct portions (12), a stabilizing tongue (10) being formed between delimiting edges (13', 13") of adjoining straight fluid duct portions (12), **characterized in that** the stabilizing tongue (10) has a delimiting edge (13', 13") extending tapering towards the arcuate fluid duct portion (34) at an angle of taper (ϕᵢ).

2. The device according to Claim 1, **characterized in that** one wall of the fluid duct (4) is formed by the covering film (3', 3") and one wall of the fluid duct (4) is formed by the delimiting edge (13', 13") of the flat frame (2).

3. The device according to Claim 1 or 2, **characterized in that** the two delimiting edges (13', 13"), extending towards the arcuate fluid duct portion (11, 34), of the stabilizing tongue define an opening angle (ϕ) in the range from 4° to 8°, preferably 6°.

4. The device according to any one of Claims 1 to 3, **characterized in that** the straight fluid duct portion (33), adjoining the inlet (35), of the fluid duct (4) has an upper wall (38'), which extends in a straight line and as an extension of the inlet (35).

5. The device according to any one of Claims 1 to 4, **characterized in that** the arcuate fluid duct portion (34) has on an outer side (39) thereof an input wall (40) and an output wall (41), which define an opening angle range (γ) ranging from 40° to 50°, preferably 45°.

6. The device according to any one of Claims 1 to 5, **characterized in that** an end (43), facing the inlet (5) and/or the outlet (6), of the rectilinear fluid duct portion (33, 33^{IV}) extends tapering towards the inlet (5) or the outlet (6) over a tapering angle range (ϕ) of 50° to 70°, preferably 60°.

7. The device according to any one of Claims 1 to 6, **characterized in that** a first covering film (3') is materially bonded with a first flat side (2') and an opposing second covering film (3") is materially bonded with a second flat side (2") of the flat frame (2).

8. The device according to any one of Claims 1 to 7, **characterized in that** the heating means is formed by two heating surfaces, wherein a first heating surface rests flat against the first covering film (3') and a second heating surface rests flat against the second covering film (3").

9. The device according to any one of Claims 1 to 8, **characterized in that** in the region of the inlet (5) and/or in the region of the outlet (6) a sensor (15), in particular a temperature sensor is arranged and/or a feed line (16).

10. The device according to any one of Claims 1 to 9, **characterized in that** a plurality of sensors (15) are arranged in one plane on an outer side of the first covering film (3') or between the first covering film (3') and the first flat side (2') of the flat frame (2).

11. The device according to any one of Claims 1 to 10, **characterized in that** the sensor has a spectrometer unit and a sensor line, wherein a first end of the sensor line is connected with the spectrometer unit and wherein a second end of the sensor line is configured such that an end face of the second end of the sensor line is positioned in a predetermined relative position directly against the outside of the covering film (3') or at a defined distance from the outside of the covering film (3').

12. The device according to Claim 11, **characterized in that** the spectrometer unit is configured as an optical reflection spectrometer unit or as an optical absorption spectrometer unit and **in that** the sensor line has at least two light guides, wherein a first light guide is used to feed light to the fluid and a second light guide is used to convey light reflected from the fluid back to the reflection spectrometer unit or absorption spectrometer unit, such that a property of the fluid can be determined in an evaluation unit on the basis of a reflection spectrum ascertained by the reflection spectrometer unit or on the basis of the absorption spectrum of the fluid ascertained by the absorption spectrometer unit.

13. The device according to any one of Claims 1 to 12, **characterized in that** the fluid housing (4) extends in one plane.

14. The device according to any one of Claims 1 to 12, **characterized in that** the fluid housing (44) extends helically with a helical fluid duct (47).

15. The device according to Claim 14, **characterized in that** the helical fluid duct (47) has flat sides, which extend perpendicular to an axis of the helical fluid housing (44).

16. The device according to Claim 14 or 15, **characterized in that** the helical fluid duct (47) is producible by adhesively bonding a covering film from a side close to the axis and a side remote from the axis to a rigid spiral skeleton.

17. The device according to any one of Claims 14 to 16, **characterized in that** the helical fluid duct is producible by welding together, with a helical weld line, two tubular covering films placed coaxially inside one another.

## Revendications

1. Dispositif destiné à chauffer des fluides en écoulement, en particulier des fluides intraveineux, avec une enveloppe à fluide plate (1) contenant un conduit de fluide (4), à travers lequel le fluide peut être guidé entre une entrée (5) et une sortie (6), avec un film de recouvrement (3', 3") et avec un moyen de chauffage, disposé à l'extérieur de l'enveloppe à fluide (1) et destiné à chauffer le fluide en écoulement à travers le conduit de fluide (4), dans lequel le conduit de fluide (4) est réalisé en forme de méandres, l'enveloppe à fluide (1) présente un cadre plat (2) avec une forme qui suit le contour du conduit de fluide (4), le cadre plat (2) présente des deux côtés d'un plan médian longitudinal un côté plat (2', 2") et les deux côtés plats (2', 2") sont reliés respectivement par liaison de matière à un film de recouvrement (3', 3") de telle sorte que des parois du conduit de fluide (4) sont formées par les deux films de recouvrement (3', 3") opposés et par des bords de limitation (13', 13") opposés du cadre plat (2), le cadre plat (2) présente une quantité de segments de conduit de fluide arqués (7, 7', 7") et une quantité de segments de conduit de fluide droits (12), dans lequel entre des bords de limitation (13', 13") de segments de conduit de fluide droits (12) adjacents se forme une languette de stabilisation (10), **caractérisé en ce que** la languette de stabilisation (10) présente, en direction du segment de conduit de fluide arqué (34), un bord de limitation (13', 13") qui se rétrécit selon un angle de rétrécissement (ϕᵢ).

2. Dispositif selon la revendication 1, **caractérisé en ce qu'**une paroi du conduit de fluide (4) est formée par le film de recouvrement (3', 3") et une paroi du conduit de fluide (4) est formée par le bord de limitation (13', 13") du cadre plat (2).

3. Dispositif selon la revendication 1 ou 2, **caractérisé en ce que** les deux bords de limitation (13', 13") de la languette de stabilisation qui s'étendent en direction du segment de conduit de fluide arqué (11, 34) fixent un angle d'ouverture (ϕ) dans la plage de 4° à 8°, de préférence 6°.

4. Dispositif selon l'une quelconque des revendications 1 à 3, **caractérisé en ce que** le segment de conduit de fluide droit (33) du conduit de fluide (4) qui se raccorde au niveau de l'entrée (35) présente une paroi supérieure (38') qui s'étend de manière rectiligne et dans le prolongement de l'entrée (35).

5. Dispositif selon l'une quelconque des revendications 1 à 4, **caractérisé en ce que** le segment de conduit de fluide arqué (34) présente au niveau d'un côté extérieur (39) une paroi d'entrée (40) et une paroi de sortie (41) qui fixent une plage d'angle d'ouverture (γ) dans la plage de 40° à 50°, de préférence 45°.

6. Dispositif selon l'une quelconque des revendications 1 à 5, **caractérisé en ce qu'**une extrémité (43) du segment de conduit de fluide droit (33, 33^{IV}) qui se trouve en regard de l'entrée (5) et/ou de la sortie (6) se rétrécit en direction de l'entrée (5) respectivement de la sortie (6) selon une plage d'angle de rétrécissement (ϕ) de 50° à 70°, de préférence 60°.

7. Dispositif selon l'une quelconque des revendications 1 à 6, **caractérisé en ce qu'**un premier film de recouvrement (3') est relié par liaison de matière à un premier côté plat (2') et un second film de recouvrement opposé (3") à un second côté plat (2") du cadre plat (2).

8. Dispositif selon l'une quelconque des revendications 1 à 7, **caractérisé en ce que** le moyen de chauffage est formé par deux surfaces de chauffage, dans lequel une première surface de chauffage se situe de manière plane au niveau du premier film de recouvrement (3') et une seconde surface de chauffage se situe de manière plane au niveau du second film de recouvrement (3").

9. Dispositif selon l'une quelconque des revendications 1 à 8, **caractérisé en ce que**, au niveau de l'entrée (5) et/ou au niveau de la sortie (6), est disposé un capteur (15), en particulier un capteur de température, et/ou un câble d'alimentation (16).

10. Dispositif selon l'une quelconque des revendications 1 à 9, **caractérisé en ce que** plusieurs capteurs (15) sont disposés dans un plan sur un côté extérieur du premier film de recouvrement (3') ou entre le premier film de recouvrement (3') et le premier côté plat (2') du cadre plat (2).

11. Dispositif selon l'une quelconque des revendications 1 à 10, **caractérisé en ce que** le capteur présente une unité de spectromètre et un câble de capteur, dans lequel une première extrémité du câble de capteur est reliée à l'unité de spectromètre et dans lequel une seconde extrémité du câble de capteur est réalisée de telle sorte qu'une surface frontale de la seconde extrémité du câble de capteur est positionnée dans une position relative prédéfinie directement sur le côté extérieur du film de recouvrement (3') ou à une distance définie du côté extérieur du film de recouvrement (3').

12. Dispositif selon la revendication 11, **caractérisé en ce que** l'unité de spectromètre est réalisée sous la forme d'une unité de spectromètre de réflexion optique ou sous la forme d'une unité de spectromètre d'absorption optique et **en ce que** le câble de capteur présente au moins deux conduits de lumière, dans lequel la lumière est amenée jusqu'au fluide par le biais d'un premier conduit de lumière et la lumière réfléchie au niveau du fluide par le biais d'un second conduit de lumière est redirigée vers l'unité de spectromètre de réflexion respectivement l'unité de spectromètre d'absorption de telle sorte que, sur la base d'un spectre de réflexion déterminé par l'unité de spectromètre de réflexion ou sur la base d'un spectre d'absorption du fluide déterminé par l'unité de spectromètre d'absorption, une propriété du fluide peut être définie dans une unité d'évaluation.

13. Dispositif selon l'une quelconque des revendications 1 à 12, **caractérisé en ce que** l'enveloppe à fluide (4) s'étend dans un plan.

14. Dispositif selon l'une quelconque des revendications 1 à 12, **caractérisé en ce que** l'enveloppe à fluide (44) s'étend en forme d'hélice avec un conduit de fluide hélicoïdal (47).

15. Dispositif selon la revendication 14, **caractérisé en ce que** le conduit de fluide hélicoïdal (47) présente des côtés plats qui s'étendent perpendiculairement à un axe de l'enveloppe à fluide hélicoïdale (44).

16. Dispositif selon la revendication 14 ou 15, **caractérisé en ce que** le conduit de fluide hélicoïdal (47) peut être fabriqué par collage d'un film de recouvrement d'un côté proche de l'axe et d'un côté distant de l'axe sur un châssis spiralé rigide.

17. Dispositif selon l'une quelconque des revendications 14 à 16, **caractérisé en ce que** le conduit de fluide hélicoïdal peut être fabriqué par soudage de deux films de recouvrement hélicoïdaux positionnés de manière coaxiale l'un dans l'autre avec une ligne de soudage hélicoïdale.
